# EUROPEAN PATENT APPLICATION

(11) **EP 2 801 356 A2**
(43) Date of publication of application: **12.11.2014**
(21) Application number: 14180286.8
(22) Date of filing: 26.06.2012
(51) Int. Cl.: A61K 31/202, A61K 47/44, A23G 1/36, A61P 27/02, A61P 17/00, A61P 9/10, A61P 25/00, A61P 9/12, A61P 1/00, A61P 3/00, A23L 1/30, A23L 2/52, A61K 36/899, A61K 35/60, A61K 36/185, A61K 36/28, A61K 36/48, A23K 1/16

(54) **DHA and EPA for the reduction of oxidative stress**

(30) Priority: 28.06.2011 EP 11171681
(62) Divisional of application: 12730510.0
(71) Applicant: Nestec S.A., 1800 Vevey (CH)
(72) Inventor: Bosco, Mohamed Nabil, 1066 Epalinges (CH); Oliveira, Manuel, 1071 CHEXBRES (CH); Destaillats, Frederic, 1077 SERVION (CH); Benyacoub, Jalil, 1066 Epalinges (CH); Brahmbhatt, Viral, 1201 Genève (CH)
(74) Representative: Stephen, Paula-Marie

(57) **Abstract**

The present invention generally relates to the prevention or treatment of disorders related to oxidative stress. For example, the present invention provides a composition for use in the prevention or treatment of oxidative stress related disorders under post-operative conditions. One embodiment of the present invention is a composition comprising DHA and EPA as active ingredients for use in the treatment or prevention of oxidative stress and/or related disorders.

## Description

The present invention generally relates to the prevention or treatment of disorders related to oxidative stress. For example, the present invention provides a composition for use in the prevention or treatment of oxidative stress related disorders under post-operative conditions. One embodiment of the present invention is a composition comprising docosahexaenoic acid (DHA) and eicosapentaenoic acid (EPA) as active ingredients for use in the treatment or prevention of oxidative stress and/or related disorders.

Intestinal damages as a result of ischemia and subsequent reperfusion plays a key role in complications of various adult surgical procedures and neonatal clinical conditions i.e. solid organ transplantation, septic shock and necrotizing enterocolitis (NEC) (reviewed in Nowicki PT et al. Clin. Perinatol., 1994 21:219-234, Carden DL et al. J. Pathol., 2009 190: 255-266 and Cerqueira NF et al. Acta Cir. Bras., 2009 24: 290-295**).** Numerous lines of evidence suggest that the generation of reactive oxygen species (ROS) and ROS-mediated damages play a central role in intestinal IR injuries.

Apart from an increase in ROS production intestinal IR leads to ROS-dependent and independent activation of and complex downstream cascades which are not well understood. The outcome of this activation is an increased expression of neutrophil adhesion molecules, inflammatory cytokines and lipid proinflammatory metabolites derived from arachidonic acid (i.e. eicosanoids). This eventually leads to mucosal injury causing increased barrier permeability which is partly responsible for distant organ damages and surgical complications.

In order to reduce oxidative stress related disorders following ischemia reperfusion, for example, authors suggest a treatment with anti-oxidants such as vitamin C (Lloberas et al., FASEB J. April 23, 2002, page 908-910).

However, vitamin C is quickly secreted from the body and may not be efficiently active unless used in repetitive and high amounts with possible side effects.

Consequently, there is a need in the art for alternative compositions that - when administered - allow to treat, prevent or alleviate disorders related to oxidative stress, in particular following ischemia reperfusion (IR).

The present inventors have addressed this need.

Hence, it was the objective of the present invention to provide the art with a composition that can be used to treat or prevent disorders related to oxidative stress, for example after ischemia reperfusion, that it easy and safe to administer, is natural and has no unwanted side effects.

The present inventors were surprised to see that they could achieve this objective by the subject matter of the independent claim. The dependant claims further develop the idea of the present invention.

The inventors provide a composition comprising docosahexaenoic acid (DHA) and eicosapentaenoic acid (EPA) that can successfully be used in the treatment, prevention or alleviation of oxidative stress and/or related disorders, for example induced by ischemia-reperfusion, and/or post-operative conditions related thereto.

Dietary intervention with omega-3 fatty acids such as eicosapentaenoic acid (EPA) and docosahexaenoic acid (DHA) has been shown to be important for brain development and for the reduction of cardiovascular disease deaths.

The current worldwide intake of EPA and DHA varies greatly and correlates well with burden of diseases that can potentially be modified by increasing EPA and DHA intake (review in Hibbeln JR et al. Am. J. Clin. Nutr., 2006 83: 1483-1493)The inventors report the effects of a dietary intervention based on a lipid blend allowing an efficient increase of systemic and intestinal EPA and DHA content after 3 weeks and conferring a protection against mucosal damages following intestinal IR injury induced by temporary occlusion of mesenteric artery.

The inventors demonstrate that increased intake of EPA and DHA can prevent mucosal damage without having an effect on neutrophil infiltration (MPO content), however, it reduces their activity (ROS).

In order to investigate the underlying mechanisms, the inventors measured changes in oxidative stress mediators (CAT & SOD) and cytokines. Quantitative analytical methodologies following intestinal IR injury and after intervention with EPA and DHA were used to characterize the impact of our dietary intervention on anti-inflammatory eicosanoid generation (e.g. 17,18-EEP and TXB3).

The results of this study show beneficial effects on mucosal damages via a reduction in oxidative stress.

Consequently, the present invention relates to a composition comprising DHA and EPA as active ingredients for use in the treatment, prevention or alleviation of oxidative stress and/or related disorders.

The present invention also concerns the use of DHA and EPA as active ingredients in the preparation of a composition for the treatment, prevention or alleviation of oxidative stress and/or related disorders.

The composition of the present invention was shown to be in particularly useful if it is for use in the treatment, prevention or alleviation of oxidative stress and/or related disorders under post operative conditions.

For example the oxidative stress and/or related disorders may be induced by ischemia-reperfusion.

Ischemia-reperfusioninjury may lead to a massive generation of free oxygen radicals, which may oxidize the cellular structures. This is counter-productive for a healing process, e.g., after an operation. The present invention allows treating, alleviating or preventing the negative impact of the resulting oxidative stress.

The composition may hence be for use under hospitalized conditions for example.

The composition was shown to be in particular effective if it contains EPA and DHA.

EPA and DHA may be obtained from any source. They may be synthesized or purified from natural compounds.

Preferably, they are provided as a natural composition or as an extract thereof. For example DHA and EPA may be provided from a lipid source comprising marine oils, such as fish oil or from microorganisms.

As fish oils often have a taste and/or smell that may be perceived as unpleasant, the fish oil or the whole lipid source may be provided in an encapsulated form.

Compounds that are known to prevent the generation of unwanted off-tastes or odors, such as lecithin or the juice of citrus fruits, for example, may be added.

The composition may comprise other lipids. For example, the lipid source may comprise cocoa butter, soybean oil, fish oil and/or sunflower oil.

For example, the lipid source may be adjusted to meet the nutritional needs of the subject to be treated. For example, the lipid source may correspond to about 7-13%, for example about 10%, of the caloric content of the composition.

The essential fatty acids, linoleic acid and alpha-linolenic acid, may correspond to about 3-5.5%, for example about 4.4%, and about 0.1-0.5%, for example about 0.3%, respectively, of the total caloric content of the composition. In addition EPA and DHA may be contained in the composition in an amount corresponding to about 0.2-0.4%, for example about 0.3%, and about 0.1-0.3%, for example about 0.2%, respectively of the total energy intake.

This may be achieved by several different oil mixtures, which all can be used and which may be selected according to taste or other preferences.

For example such a lipid source may comprise about 5-10 weight-% cocoa butter, about 45-55 weight-% soybean oil, about 15-25 weight-% fish oil and about 20-25 weight-% sunflower oil.

The lipid source may comprise about 7 weight % cocoa butter, about 50 weight-% soybean oil, about 20 weight-% fish oil and about 23 weight-% sunflower oil.

Typically, such a lipid source comprises a mixture of saturated fatty acids, monounsaturated fatty acids and polyunsaturated fatty acids.

The amounts of these fatty acids may be adjusted based on the needs of the subject to be treated.

For example, the lipid source may comprise about 18-22 weight-% saturated fatty acids, about 20-25 weight-% monounsaturated fatty acids and about 50-55 weight-% polyunsaturated fatty acids.

Oxidative damage is often mediated by reactive oxygen species. Such reactive oxygen species may be free radicals, for example.

Consequently, the oxidative stress related disorder may be linked to the presence of free radicals in a body such as •O²⁻, the superoxide anion; H₂O₂, hydrogen peroxide; •OH, the hydroxyl radical; ROOH, organic hydroperoxide; RO•, alkoxy radicals; ROO•, peroxy radicals; HOCl, hypochlorous acid; OONO⁻, peroxynitrite; and/or NO•.

Many organs can be either directly or indirectly damaged by oxidative stress. Indirect damage is often seen in remote organs following reperfusion. Such indirect damage is problematic as it can lead, e.g., to a multiple organ disturbance syndrome following intestine ischemia reperfusion. Oftentimes the lung may be affected by indirect damage.

The composition of the present invention reduces the risk for such direct or indirect damage of organs and may consequently be used in the treatment or prevention of oxidative damage to intestine, liver, lung, heart, kidney, and/or skin.

The oxidative stress related disorder may be any such disorder.

For example, the oxidative stress related disorder may be selected from the group consisting of an inflammatory response to oxidative stress, retinal degeneration, mitohormesis, atherosclerosis, amyotrophic lateral sclerosis (ALS), multiple sclerosis (MS), Friedreich's ataxia, tardive dyskynesia, brain injuries such as ischemia, skin photoaging, reperfusion injury or stroke, myocardial infarction, hypertension, heart failure, epilepsy, hyperhomocysteinemia, physiological senescence, sepsis, stress following organ transplantation or combinations thereof.

The inventors have demonstrated experimentally by means of example using a rodent model that the composition of the present invention can very successfully be used to limit intestinal damages following ischemia-reperfusion (IR), e.g., in the splanchnic area. Consequently, in particular visceral organs may be protected by the composition of the present invention.

The effectiveness of the composition of the present invention follows a dose-response curve.

In therapeutic applications, compositions are administered in an amount sufficient to at least partially cure or arrest the symptoms of the disease and its complications. An amount adequate to accomplish this is defined as "a therapeutically effective dose". Amounts effective for this purpose will depend on a number of factors known to those of skill in the art such as the severity of the disease and the weight and general state of the patient.

In prophylactic applications, compositions according to the invention are administered to a patient susceptible to or otherwise at risk of a particular disease in an amount that is sufficient to at least partially reduce the risk of developing a disease. Such an amount is defined to be "a prophylactically effective dose". Again, the precise amounts depend on a number of patient specific factors such as the patient's state of health and weight.

The compositions of the present invention may be administered in a a therapeutically effective dose or a prophylactically effective dose. Skilled artisans will be able to determine these dosages based on the information provided herein and the state of the patient.

For example, the composition may be to de administered in an amount corresponding to a combined daily dosage of EPA and DHA are of at least 400 mg.

EPA and DHA may be provided in any ratio, for example in a weight ratio in the range of 10:1 to 1:10, for example 7:1 to 1:1, e.g. about 5:1 to 1:1.

Preferably, EPA and DHA are comprised in the composition in a weight ratio in the range of about 2:1 to 1:1. Very good results were obtained with a weight ratio of EPA and DHA in the range of about 3:2.

In clinical settings the compositions may be to be administered in amounts corresponding to a combined daily intake of EPA and DHA of about 2-5 g, for example, 3-4 g.

For best results it is advisable to administer the compositions of the present invention consecutively over a period of several days.

If an operation is planned, and in particular if an ischemia-reperfusion is foreseen, the administration may begin for example 7 days, 3 days, 2 days or 1 day before the operation as preventive measure.

For example, the administration may begin 3 to 7 days before an intervention.

The administration may also be started immediately after the operation and/or ischemia-reperfusion.

The composition may be to be administered daily over a period of at least 3 days, for example at least a week or at least two weeks.

The composition of the present invention may be to be administered to any patient. The patient may be a human or animal patient. The animal may be a companion animal such as a dog or a cat.

The compositions may be to be administered topically, orally, enterally or parenterally.

Typically, the compositions will be administered orally with the normal food intake.

Should the normal food intake be not possible, the composition may be to be administered enterally, e.g., via tube feeding, or in severe cases, where enteral administration is not possible or not advised parenterally.

In cases of parenteral administration the composition usually does not contain a carbohydrate source, but carbohydrates may be administered separately, via a glucose drip, for example.

Typically, however, the composition may be administered as a food product or as a drinkable composition.

As such, the composition may be selected from the group consisting of a food product, a pet food product, a nutraceutical, a drink, a food supplement, a powdered nutritional formula to be reconstituted in milk or water, or a medicament.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples.

### Examples:

8 week old male Sprague-Dawley rats (Charles-River - France) were used. Animals were received two weeks prior to dietary intervention for acclimatation in our animal facility. All animals were kept under 12 h light/dark cycles at constant room temperature in specific pathogen-free conditions. They had free access to a commonly used basal diet (AIN-93M) and tap water.

After two weeks, rats were randomly assigned to one of three different experimental groups (n=8 per group) i.e. (i) Sham group- this group was provided control diet for 3 weeks and underwent a sham surgical procedure; (ii) I/R group- this group was provided control diet for 3 weeks followed by I/R surgical protocol and (iii) I/R+ NRC lipid blend group- this group was provided diet supplemented with a lipid blend (described below) for 3 weeks and underwent I/R surgical protocol at the end of dietary intervention.

At the end of the third week under control or NRC supplemented diets, animals underwent either a sham procedure or an I/R surgical protocol, which consists of a 30 min ischemic period followed by a 3h reperfusion period (as described in Yoshida N. et al. Mol Med Report, 2011 4:81-86). At the end of the reperfusion period, animals were sacrificed then sera and organs were collected.

Maintenance AIN-93M diet contains 10% of total energy provided as fat. The essential fatty acids, linoleic acid and alpha-linolenic acid provide ∼ 4.4% and 0.3% of the total energy intake respectively. In the NRC interventional diet, we kept these values as close as possible to control with a specific blend described below. In addition EPA and DHA while absent in the control diet are provided in the supplemented diet in amounts that are close to fish oil, ∼ 0.3% and 0.2% of total energy intake, respectively.

Total small intestine protein, superoxide dismutase, catalase, nitrate / nitrite, MPO content or activities were measured with commercially available kits (from Roche, Sigma or Cayman). EPA and DHA incorporation were measured with standard HPLC methods and adequate commercially available standards. Eicosanoids were quantified by HPLC-MS/MS with adequate commercially available standards as described in Pouteau E. et al. Nutr Metab, 2010 7:8.

All the data presented herein are expressed as mean ± SEM. Comparison of the three groups was made using Student's t-test for unpaired data when appropriate. Differences were considered statistically significant for P value <0.05.

### Results

**Table 1a and b give the lipid blend description and nature of fatty acid contained.**

| a. | | |
|---|---|---|
| **Lipid blend composition** | **Control (% of total fat)** | **NRC blend (% of total fat)** |
| Corn oil | 35 | 0 |
| Cocoa butter | 15 | 7 |
| Soybean oil | 50 | 50 |
| Fish oil | 0 | 20 |
| Sunflower oil | 0 | 23 |

| b. | | |
|---|---|---|
| **Lipid blend composition** | **Control (% of total fat)** | **NRC blend (% of total fat)** |
| SFA | 21.7 | 20.1 |
| MUFA | 26 | 23.2 |
| PUFA | 52.1 | 52.9 |
| Other | 0.2 | 3.8 |

**Table 2 summarizes biochemical measured made with animals (n=8/group).**

| **Assays** | **SHAM** | **I/R** | **I/R + lipid blend** |
|---|---|---|---|
| | | | **(* when statistically different from I/R group)** |
| Total protein (ug/mg of intestine tissue) | 4.88 ±0.26 | 4.45 ±0.27 | 4.90 ±0.36 * |
| SOD Units /100 mg intestine tissue | 408.4 ±18.9 | 371.4 ±41.6 | 476.4 ±16.5 * |
| CAT umol/min/100 mg intestine tissue | 380 ±66.9 | 372.6 ±62.1 | 482.3 ±90.6 * |
| Nitrate / Nitrite uM/g intestine tissue | 27.4 ±19.5 | 37.6 ±29.5 | 13.7 ±9 * |
| MPO ng/mg intestine tissue | 43.3 ±9.3 | 98.1 ±36.6 | 103.7 ±32 |
| ROS (MFI) Neutrophil mean fluoresence intensity | 41.3 ±5.5 | 47.8 ±3.7 | 42.5 ±4.8 |
| Non-esterified EPA (ng/mg tissue) | 95.2±44.8 | 69.8±27.1 | 492.3 ±240.6 * |
| Non-esterified DHA (ng/mg tissue) | 73.2±28.3 | 69.6±23.3 | 152.2 ±69.6 * |
| 17,18-EEP (ng/mg tissue) | 0.3±0.2 | 0.2±0.1 | 3.1 ±2.1 * |
| TXB3 (ng/mg tissue) | 0.14±0.08 | 0.14±0.03 | 0.50 ±0.37 * |

### Conclusions

Our study demonstrate that our particular lipid blend enriched with omega-3 fatty acids can (e.g., when incorporated in a food matrix) limit e.g. intestinal oxidative stress related damages following ischemia-reperfusion (IR) in the splanchnic area. Interestingly, NRC lipid blend fed animals display a higher expression of enzymes of the oxidative stress machinery and lipidomic analyses of intestinal tissue clearly show global increase of antiinflammatory lipid metabolites. The present results show for the first time how a dietary intervention can modulate eicosanoid formation and limit events associated to IR damages.

## Claims

1. Composition comprising DHA and EPA as active ingredients for use in the treatment, prevention or alleviation of oxidative stress and/or related disorders induced by ischemia-reperfusion.

2. Composition for use in accordance with claim 1 comprising EPA and DHA in a weight ratio in the range of about 2:1 to 1:1, preferably about 3:2.

3. Composition for use in accordance with one of the preceding claims wherein DHA and EPA are provided from a lipid source comprising marine oils, such as fish oil.

4. Composition for use in accordance with claim 3, wherein the lipid source comprises cocoa butter, soybean oil, fish oil and sunflower oil.

5. Composition for use in accordance with claim 4, wherein the lipid source comprises about 5-10 weight-% cocoa butter, about 45-55 weight-% soybean oil, about 15-25 weight-% fish oil and about 20-25 weight-% sunflower oil.

6. Composition for use in accordance with claim 3, wherein the lipid source comprises about 18-22 weight-% saturated fatty acids, about 20-25 weight-% monounsaturated fatty acids and about 50-55 weight-% polyunsaturated fatty acids.

7. Composition for use in accordance with one of the preceding claims, wherein the oxidative stress related disorder is linked to the presence of free radicals in a body such as •O²⁻, the superoxide anion; H₂O₂, hydrogen peroxide; •OH, the hydroxyl radical; ROOH, organic hydroperoxide; RO•, alkoxy radicals; ROO•, peroxy radicals; HOCl, hypochlorous acid; OONO⁻, peroxynitrite; and/or NO•.

8. Composition for use in accordance with one of the preceding claims in the treatment or prevention of oxidative damage to intestine, liver, lung, heart, kidney, and/or skin.

9. Composition for use in accordance with one of the preceding claims to limit intestinal damages following ischemia-reperfusion (IR), e.g., in the splanchnic area.

10. Composition for use in accordance with one of claims 1 to 8, wherein the oxidative stress related disorder is selected from the group consisting of an inflammatory response to oxidative stress, retinal degeneration, mitohormesis, atherosclerosis, amyotrophic lateral sclerosis (ALS), multiple sclerosis (MS), Friedreich's ataxia, tardive dyskynesia, brain injuries such as ischemia, skin photoaging, reperfusion injury or stroke, myocardial infarction, hypertension, heart failure, epilepsy, hyperhomocysteinemia, physiological senescence, sepsis, stress following organ transplantation or combinations thereof.

11. Composition for use in accordance with one of the preceding claims, wherein EPA and DHA are provided in a daily dose of at least 400 mg.

12. Composition for use in accordance with one of the preceding claims, wherein the composition is to be administered daily over a period of at least 3 days, for example at least a week or at least two weeks.

13. Composition for use in accordance with one of the preceding claims, to be administered to human or pets, for example companion animals such as dogs or cats.

14. Composition for use in accordance with one of the preceding claims to be administered topically, orally, enterally or parenterally.

15. Composition for use in accordance with one of the preceding claims wherein the composition is selected from the group consisting of a food product, a pet food product, a nutraceutical, a drink, a food supplement, a powdered nutritional formula to be reconstituted in milk or water, or a medicament.
